(19) Europäisches Patentamt / European Patent Office / Office européen des brevets

(11) **EP 4 764 500 A1**

(12) **EUROPEAN PATENT APPLICATION**
published in accordance with Art. 153(4) EPC

(43) Date of publication:
**24.06.2026 Bulletin 2026/26**

(21) Application number: **23948805.9**

(22) Date of filing: **14.08.2023**

(51) International Patent Classification (IPC):
**G01N 33/68** (2006.01)

(52) Cooperative Patent Classification (CPC):
**G01N 33/68**

(86) International application number:
**PCT/CN2023/112993**

(87) International publication number:
**WO 2025/035370 (20.02.2025 Gazette 2025/08)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Shenzhen Mindray Bio-Medical
Electronics Co., Ltd.
Shenzhen, Guangdong 518057 (CN)**

(72) Inventors:
• **LIU, Yuqing
  Shenzhen, Guangdong 518057 (CN)**

• **ZHANG, Yi
  Shenzhen, Guangdong 518057 (CN)**
• **LIU, Junjun
  Shenzhen, Guangdong 518057 (CN)**
• **KATRUKHA, Ivan
  Moscow 117186 (RU)**
• **BEREZNIKOVA, Anastasia
  Moscow 117186 (RU)**

(74) Representative: **KIPA AB
Drottninggatan 11
252 21 Helsingborg (SE)**

(54) **COMPOSITION, KIT AND METHOD FOR DETECTING TOTAL CTNITC, AND USE THEREOF**

(57) The present application relates to a composition, a kit and a method for detecting total cTnITC, and the use thereof. Specifically, the present application relates to a composition and a kit containing an antibody that specifically binds to any one segment of a cTnT amino acid sequence at positions 223-287 and an antibody that specifically binds to any one segment of a TnC amino acid sequence, a detection method, and the use in in-vitro diagnosis of myocardial damage.

FIG. 9

**Description**

TECHNICAL FIELD

The disclosure relates to the field of immunoassays, and in particular relates to a composition and a kit for detecting a total cardiac troponin ternary complex cTnITC, a detection method, and the use thereof in the *in-vitro* diagnosis of myocardial injury.

BACKGROUND

Cardiac troponin (cTn) is a specific marker for myocardial injury and may be used to detect myocardial injury after or during myocardial infarction. Troponin is composed from three subunits, namely cardiac troponin I (cTnI), cardiac troponin T (cTnT) and troponin C (TnC). The concentrations of cTnI and cTnT in serum are highly correlated with the severity of myocardial injury, and are recommended by the American and European Cardiology Societies as highly specific and sensitive markers for myocardial infarction. However, numerous factors induce elevated cardiac troponin concentrations in the blood, such as pulmonary embolism, acute or chronic heart failure, cardiac trauma, endocarditis and myocarditis, in addition to myocardial infarction. This reduces the clinical specificity of cTn for myocardial infarction. Therefore, a combination with other methods is typically required for the diagnosis of myocardial infarction.

Cardiac troponin typically binds to actin filaments in the form of a ternary complex cTnITC. In the event of myocardial injury, troponin dissociates from myofilaments and is released into the bloodstream. Intracellularly or in the circulating blood, full-length cTnITC undergoes proteolytic degradation, gradually forming low-molecular-weight ternary complexes (LMW-cTnITC), binary complexes (cTnIC) and free cTnT. Studies have shown that the existing forms of troponin in the blood are associated with the physiological and pathological status of individuals. Detection of the troponin ternary complex cTnITC helps physicians to gain a more comprehensive understanding of the physiological and pathological status of patients, guiding patient prognosis.

Currently, the traditional immunological methods for cardiac troponin detection mainly include enzyme-linked immunosorbent assay (ELISA), gold immunochromatographic assay (GICA), electrochemiluminescence (ECL), chemiluminescence immunoassay (CLIA), and other technologies. The chemiluminescence immunoassay technology has high sensitivity, strong specificity, a wide linear range, good operability and high degree of automation, and is the immunological assay method with the broadest clinical application prospects. Based on the chemiluminescence immunoassay technology, existing high-sensitivity cardiac troponin detection methods are typically used to detect cTnI and cTnT, but cannot distinguish between troponin complexes and free troponin. It has been reported in the literature that a double-antibody sandwich immunoassay for cTnITC complexes is achieved by using capture antibodies that recognise the epitopes of the binary TnIC complex and detection antibodies that recognise the TnT antigen. This detection method has a low signal-to-noise ratio and insufficient specificity, making it difficult to achieve rapid, sensitive and specific detection of the total content of the ternary cTnITC complex. Therefore, developing a highly sensitive and rapid detection kit for detecting the total amount of the cardiac troponin ternary complex cTnITC (i.e., the total content of cTnITC) in the blood is of particular importance for the diagnosis and prognostic guidance of cardiovascular diseases.

SUMMARY

An object of the disclosure is to provide a technical solution capable of detecting the total content of a cardiac troponin ternary complex cTnITC in a sample with high sensitivity and rapid speed. By means of the cooperation of a capture antibody and a detection antibody, the specific detection of total cTnITC is achieved. In particular, the addition of an antibody that specifically binds to TnC to the capture antibody or detection antibody is very beneficial for increasing the signal-to-noise ratio of the detection system and improving the detection sensitivity for cTnITC.

In a first aspect, the disclosure provides a composition for detecting a cardiac troponin ternary complex in a sample, comprising a first group of antibodies and a second group of antibodies, wherein

the first group of antibodies comprises one or more antibody 1, each being independently selected from antibodies that specifically bind to any one segment of the amino acid sequence of positions 223-287 of cTnT;
the second group of antibodies comprises one or more antibody 2, each being independently selected from antibodies that specifically bind to any one segment of the amino acid sequence of TnC.

In a second aspect, the disclosure provides a kit for detecting a cardiac troponin ternary complex in a sample, comprising a capture antibody and a detection antibody, wherein the capture antibody is selected from one group of a first group of antibodies and a second group of antibodies, and the detection antibody is selected from the other group of the first group of antibodies and the second group of antibodies, wherein

the first group of antibodies comprises one or more antibody 1, each being independently selected from antibodies that specifically bind to any one segment of the amino acid sequence of positions 223-287 of cTnT;

the second group of antibodies comprises one or more antibody 2, each being independently selected from antibodies that specifically bind to any one segment of the amino acid sequence of TnC.

In a third aspect, the disclosure provides a method for detecting a cardiac troponin ternary complex in a sample *in vitro,* comprising steps of:

obtaining a test sample;

contacting the test sample with a capture antibody to form an antibody-antigen complex;

contacting the antibody-antigen complex with a detection antibody bearing a detectable label to form an antibody-antigen-antibody complex; and

detecting the signal produced by the detectable label to determine the presence and/or total amount of a cardiac troponin ternary complex in the test sample,

wherein the capture antibody is selected from one group of a first group of antibodies and a second group of antibodies, and the detection antibody is selected from the other group of the first group of antibodies and the second group of antibodies:

the first group of antibodies comprises one or more antibody 1, each being independently selected from antibodies that specifically bind to any one segment of the amino acid sequence of positions 223-287 of cTnT;

the second group of antibodies includes one or more antibody 2, each being independently selected from antibodies that specifically bind to any one segment of the amino acid sequence of TnC.

In a fourth aspect, the disclosure provides the use of the composition according to any of the first aspect in the preparation of a reagent for detecting a cardiac troponin ternary complex.

In a fifth aspect, the disclosure provides the use of the composition according to any of the first aspect, the kit according to any of the second aspect, or the method according to any of the third aspect in the *in-vitro* diagnosis of myocardial injury.

In a sixth aspect, the disclosure provides a method for diagnosing myocardial injury *in vitro,* comprising a step of detecting a cardiac troponin ternary complex in a sample from a subject using the composition according to any of the first aspect, the kit according to any of the second aspect, or the method according to any of the third aspect.

BRIEF DESCRIPTION OF THE DRAWINGS

The drawings illustrated herein are intended to provide a further understanding of the disclosure and form a part hereof, and exemplary embodiments of the disclosure and the description thereof are intended to explain the disclosure and are not to be construed as unduly limiting the disclosure. In the figures:

FIG. 1 shows the signal-to-noise ratio analysis of total cTnITC detection kits;

FIG. 2 shows the verification of specificity of total cTnITC detection kits using serum samples;

FIG. 3 shows the linear analysis of total cTnITC detection kit 3;

FIG. 4 shows the linear analysis of total cTnITC detection kit 4;

FIG. 5 shows the signal-to-noise ratio analysis of total cTnITC detection kit 3 and kit 3a;

FIG. 6 shows the detection signal-to-noise ratio analysis of total cTnITC detection kit 3 and kit 3a in clinical serum samples;

FIG. 7 shows the verification of specificity of total cTnITC detection kit 3a using serum samples;

FIG. 8 shows the analysis of trends of change in total cTnITC detection using total cTnITC detection kits, and in high-sensitivity cTnI and cTnT detection using existing kits; and

FIG. 9 shows the analysis of rates of change in concentrations of total cTnITC detection using total cTnITC detection kits, and of high-sensitivity cTnI and cTnT detection using existing kits.

DESCRIPTION OF THE PREFERRED EMBODIMENTS

The technical solutions in the embodiments of the disclosure will be described below clearly and completely in conjunction with the drawings in the embodiments of the disclosure. Obviously, the embodiments described are merely some of, rather than all of the embodiments of the disclosure. The following description of at least one exemplary embodiment is merely illustrative in nature and in no way construed as limiting the disclosure and the application or use thereof. On the basis of the embodiments in the disclosure, all the other embodiments that would have been obtained by those of ordinary skill in the art without any inventive effort shall fall within the scope of protection of the disclosure.

Herein, unless otherwise stated, the scientific and technical terms used have the meanings commonly understood by those skilled in the art. Moreover, the immunological laboratory procedures used herein are conventional procedures widely used in the respective fields. Meanwhile, in order to facilitate a better understanding of the embodiments of the disclosure, definitions and explanations of relevant terms are provided below.

As used herein, the terms "comprise", "include" or any other variation thereof are intended to cover non-exclusive inclusion, so that a method or an apparatus comprising a series of elements comprises not only explicitly recorded elements, but also other elements not explicitly listed, or elements inherent in implementing the method or apparatus. In the absence of more restrictions, the element defined by the phrase "comprise a/an..." does not exclude another related element in a method or device that includes the element.

As used herein, the term "at least one" refers to one or more than one under reasonable conditions, for example, two, three, four, five, or ten, etc.

As used herein, the terms "first" and "second" are only used to distinguish similar objects, and do not represent a specific order for the objects. It may be understood that "first" and "second" may be interchanged in a specific order or sequence where permissible. It should be understood that the objects distinguished by "first" and "second" may be interchanged where appropriate, so that the embodiments of the disclosure described herein can be implemented in an order other than those illustrated or described herein.

As used herein, the term "specific binding" refers to a non-random binding reaction between two molecules (i.e., binding molecule and target molecule), such as a reaction between an antibody and the antigen to which it is directed. The binding affinity between the two molecules can be described by the KD value. The KD value refers to the dissociation constant derived from the ratio of kd (the dissociation rate of a specific binding molecule-target molecule interaction; also referred to as koff) to ka (the association rate of a specific binding molecule-target molecule interaction; also referred to as kon), or expressed as kd/ka in molar concentration (M). The smaller the KD value, the tighter the binding between the two molecules and the higher the affinity. In certain embodiments, an antibody that specifically binds to an antigen (or an antibody specific for an antigen) refers to an antibody that binds to the antigen with a KD of less than about $10^{-5}$ M, for example, less than about $10^{-6}$ M, $10^{-7}$ M, $10^{-8}$ M, $10^{-9}$ M or $10^{-10}$ M or less. Corresponding methods for analyzing antibody specificity are described, for example, in the following references: Harlow & Lane (1988) Antibodies: A Laboratory Manual, Cold Spring Harbor Laboratory Press, and Harlow & Lane (1999) Using Antibodies: A Laboratory Manual, Cold Spring Harbor Laboratory Press. Nonlimiting examples of suitable studies include, for example, binding studies, and blocking and competition studies performed with molecules that are structurally and/or functionally closely related. These studies can be performed using the following methods, for example, fluorescence-activated cell sorting (FACS) analysis, flow cytometry titration (FACS titration) analysis, surface plasmon resonance (SPR), isothermal titration calorimetry (ITC), fluorescence titration or radiolabelled ligand binding assay. Further methods include, for example, Western Blot, ELISA (including competitive ELISA) testing, RIA testing, ECL testing and IRMA testing.

As used herein, the term "troponin (Tn)" refers to a protein located on actin filaments within muscle cells that regulates the calcium-mediated interaction between actin and myosin. Troponin is present in cardiac and skeletal muscles and is composed of three subunits: troponin T (TnT), troponin I (TnI) and troponin C (TnC). TnT is a tropomyosin-binding subunit that interacts with actin and tropomyosin; TnI is an inhibitory subunit that inhibits the ATPase activity of actomyosin; and TnC is the only subunit that binds to calcium and can mediate the contraction of skeletal or cardiac muscles. The term "cardiac troponin (cTn)" refers to all troponin isoforms expressed in cardiac cells, preferably subendocardial cells. These isoforms have been well characterised in the art, for example, as described in Anderson 1995, Circulation Research, vol. 76, no. 4: 681-686 and Ferrieres 1998, Clinical Chemistry, 44: 487-493. The term "cardiac troponin" further includes variants of a specific cardiac troponin that have at least the same basic biological and immunological properties as the specific cardiac troponin. In particular, these variants are considered to share the same basic biological and immunological properties if they are detected by the same specificity as mentioned herein. It should be understood that troponin isoforms may be determined collectively (simultaneously or sequentially) or individually (i.e., without assaying any other isoforms whatsoever).

As used herein, the term "cardiac troponin T (cTnT)" refers to the cardiac troponin T subunit, the amino acid sequence of which is disclosed in the UniProt database under accession number P45379.

As used herein, the term "cardiac troponin I (cTnI)" refers to the cardiac troponin I subunit, the amino acid sequence of which is disclosed in the UniProt database under accession number P19429.

As used herein, the term "troponin C (TnC)" refers to the troponin C subunit, the amino acid sequence of which is disclosed in the UniProt database under accession number P63316.

As used herein, the terms "total cardiac troponin ternary complex" and "total cTnITC" are used interchangeably herein and are intended to encompass complexes formed by all full-length proteins or fragments of TnC, full-length proteins or fragments of cTnI, one or more fragments within amino acid residues 223-287 of cTnT, and optionally one or more fragments within amino acid residues 1-222 of cTnT.

As used herein, the term "antibody" has the meaning commonly understood in the art and refers to an immunoglobulin molecule that is generally composed of two pairs of polypeptide chains, each pair containing one light chain (LC) and one heavy chain (HC). Antibody light chains may be classified as κ (kappa) and λ (lambda) light chains. Heavy chains may be classified as μ, δ, γ, α and ε heavy chains, which define the antibody isotypes as IgM, IgD, IgG, IgA and IgE, respectively. Each heavy chain is composed of a heavy chain variable region (VH) and a heavy chain constant region (CH). Each light chain is composed of a light chain variable region (VL) and a light chain constant region (CL). The light chain constant region is composed of a single domain CL. The constant domains are not directly involved in the binding of an antibody to an antigen, but exhibit a variety of effector functions. For example, these domains can mediate the binding of immunoglobulins to host tissues or factors, including various cells of the immune system (e.g., effector cells) and the first component of the classical complement system (C1q). The VH and VL regions may be further subdivided into hypervariable regions (referred to as complementarity-determining regions, CDRs), interspersed with more conserved regions referred to as framework regions (FRs). Each VH and VL is composed of three CDRs and four FRs arranged from the amino terminus to the carboxyl terminus in the following order: FR1, CDR1, FR2, CDR2, FR3, CDR3, and FR4. The variable regions (VH and VL) of each heavy/light chain pair form an antigen-binding site, respectively. The assignment of amino acids to each region or domain may follow the definitions described in Kabat, Sequences of Proteins of Immunological Interest (National Institutes of Health, Bethesda, Md. (1987 and 1991)), or Chothia & Lesk (1987), J. Mol. Biol. 196: 901-917; Chothia et al. (1989), Nature 342: 878-883. As used herein, unless otherwise clearly defined in the context, when referring to the term "antibody", it includes not only intact antibodies but also antigen-binding fragments of antibodies.

The disclosure is applicable to various detection systems including, but not limited to, fluorescence-labelled reaction systems, enzyme-linked immunosorbent assay (ELISA) systems, bioluminescent systems, etc.

Detection reagent and method

An object of the disclosure is to provide a detection reagent and a method capable of sensitively detecting the total content of various forms of cardiac troponin ternary complex cTnITC in a sample, and specifically distinguishing cTnITC from other forms of cTn, including cTnT, cTnI and cTnIC.

In particular, in a first aspect, the disclosure provides a composition for detecting a cardiac troponin ternary complex in a sample, which composition includes a first group of antibodies and a second group of antibodies, wherein

the first group of antibodies includes one or more antibody 1, each being independently selected from antibodies that specifically bind to any one segment of the amino acid sequence of positions 223-287 of cTnT;
the second group of antibodies includes one or more antibody 2, each being independently selected from antibodies that specifically bind to any one segment of the amino acid sequence of TnC.

On the basis of the first aspect, a second aspect of the disclosure provides a kit for detecting a cardiac troponin ternary complex in a sample, which kit includes a capture antibody and a detection antibody, where the capture antibody is selected from one group of a first group of antibodies and a second group of antibodies, and the detection antibody is selected from the other group of the first group of antibodies and the second group of antibodies, wherein

the first group of antibodies includes one or more antibody 1, each being independently selected from antibodies that specifically bind to any one segment of the amino acid sequence of positions 223-287 of cTnT;
the second group of antibodies includes one or more antibody 2, each being independently selected from antibodies that specifically bind to any one segment of the amino acid sequence of TnC.

That is, the capture antibody includes at least antibody 1 and the detection antibody includes at least antibody 2; alternatively, the capture antibody includes at least antibody 2 and the detection antibody includes at least antibody 1.

In some embodiments, the capture antibody may be coated on the surface of a solid-phase carrier (e.g., magnetic beads, latex particles, microplates, plastic beads, plastic tubes, immunochromatographic test strips or detection cards) to capture an antigen in a sample. Subsequently, a detection antibody bearing a detectable label is allowed to bind to the capture antibody coated on the surface of the solid-phase carrier, and the content of the antigen in the sample is determined by measuring the amount of the detectable label on the bound detection antibody.

The detectable label may be any substance detectable by fluorescent, spectroscopic, photochemical, biochemical, immunological, electrical, optical or chemical means. Particularly preferably, such labels are suitable for immunological detection (e.g., enzyme-linked immunoassay, radioimmunoassay, fluorescence immunoassay, chemiluminescent immunoassay, etc.). Such labels are well known in the art and include, but are not limited to, enzymes (e.g., horseradish peroxidase, alkaline phosphatase, β-galactosidase, urease, glucose oxidase, etc.), radionuclides (e.g., 3H, 125I, 35S, 14C or 32P), fluorescent dyes (e.g., fluorescein isothiocyanate (FITC), fluorescein, tetramethylrhodamine isothiocyanate (TRITC), phycoerythrin (PE), Texas Red, rhodamine, quantum dots or cyanine dye derivatives (e.g., Cy7 and Alexa 750)), chemiluminescent substances (such as acridine ester compounds), and biotin for binding to avidin (e.g., streptavidin) modified with the above labels. The labels encompassed in the disclosure may be detected by methods known in the art. For example, radiolabels may be detected using photographic film or a scintillation counter, and fluorescent markers may be detected using a light detector to detect the emitted light. Enzyme labels are typically detected by providing the enzyme with a substrate and detecting the reaction products produced by the action of the enzyme on the substrate. Chemiluminescent substances (e.g., acridinium ester compounds) are typically detected by providing an excitation solution and/or a catalyst to the luminescent substance to measure the emitted light. Biotin is typically detected by providing avidin (e.g., streptavidin) modified with the above labels for the biotin and detecting the labels carried by the avidin bound to the biotin. In certain embodiments, the detectable labels as described above may be linked to the antibody or the antigen-binding fragment thereof of the disclosure via a linker of different lengths to reduce potential steric hindrance. In some embodiments, the detectable labels are selected from fluoresceins, chemiluminescent substances (e.g., acridinium ester compounds), enzymes (e.g., horseradish peroxidase or alkaline phosphatase), radioisotopes, biotin, colloidal gold and magnetic particles.

In some embodiments, the kit may further include a reagent for enabling the detection of a corresponding detectable label (a substrate for the detectable label). For example, when the detectable label is an enzyme, the kit may further include a chromogenic substrate for the corresponding enzyme, such as o-phenylenediamine (OPD), tetramethylbenzidine (TMB), ABTS or luminol compounds for horseradish peroxidase, or p-nitrophenyl phosphate (p-NPP) or AMPPD for alkaline phosphatase. For example, when the detectable label is a chemiluminescent reagent (e.g., acridinium ester compound), the kit may further include a pre-excitation solution and/or an excitation solution for chemiluminescence.

The kit of the disclosure is particularly suitable for immunoassays, such as ELISA, and particularly the double-antibody sandwich chemiluminescent immunoassay.

In the disclosure, the amount of total cTnITC may be detected directly or indirectly. The amount or concentration of the detection antibody may be directly detected on the basis of the detectable label signal thereof, which signal is directly correlated with the target antigen in the sample. Such signals are sometimes referred to herein as intensity signals. For example, such signals may be obtained by measuring the intensity values of the specific physical or chemical properties of the detection antibody. Indirect measurements include measurement of signals from secondary components (i.e., not the detection antibody itself) or biological readout systems, such as measurable cellular responses, ligands, labels or enzymatic reaction products.

In a third aspect, the disclosure provides a method for detecting a cardiac troponin ternary complex in a sample *in vitro,* which method includes the steps of:

obtaining a test sample;
contacting the test sample with a capture antibody to form an antibody-antigen complex;
contacting the antibody-antigen complex with a detection antibody bearing a detectable label to form an antibody-antigen-antibody complex; and
detecting the signal produced by the detectable label to determine the presence and/or total amount of a cardiac troponin ternary complex in the test sample,
wherein the capture antibody is selected from one group of a first group of antibodies and a second group of antibodies, and the detection antibody is selected from the other group of the first group of antibodies and the second group of antibodies:

the first group of antibodies includes one or more antibody 1, each being independently selected from antibodies that specifically bind to any one segment of the amino acid sequence of positions 223-287 of cTnT;
the second group of antibodies includes one or more antibody 2, each being independently selected from antibodies that specifically bind to any one segment of the amino acid sequence of TnC.

In the first aspect to the third aspect of the disclosure, as some embodiments, the first group of antibodies further includes one or more antibody 3, each being independently selected from antibodies that specifically bind to any one segment of the amino acid sequence of positions 67-222 of cTnT.

Herein, by means of combining the antibody 1 with an antibody that specifically recognises amino acids at positions 67-222 of cTnT, the signal-to-noise ratio of the detection reagent can be significantly increased.

In the first aspect to the third aspect of the disclosure, as some embodiments, the second group of antibodies further includes:

one or more antibody 4, each being independently selected from antibodies that specifically bind to cTnIC; and/or
one or more antibody 5, each being independently selected from antibodies that specifically bind to any one segment of the amino acid sequence of cTnI at positions 18-210.

In the first aspect to the third aspect of the disclosure, as some embodiments, the first group of antibodies includes one or more of the antibody 1 and one or more of the antibody 3; the second group of antibodies includes one or more of the antibody 2, and one or more of the antibody 4 and/or one or more of the antibody 5.

In some embodiments, the first group of antibodies includes one antibody 1, and optionally one antibody 3. The second group of antibodies includes one antibody 2, and optionally one antibody 4 and/or one antibody 5.

In some embodiments, the first group of antibodies includes at least two antibody 1. In some embodiments, the second group of antibodies includes at least two antibody 2. In some embodiments, the first group of antibodies includes at least two antibody 1, and optionally at least two antibody 3; the second group of antibodies includes at least two antibody 2, and optionally at least two antibody 4 and/or at least two antibody 5. A combination of multiple antibodies that bind to the same target protein is beneficial for the detection of total cTnITC.

The binding epitopes of the antibodies are screened in the disclosure. In some embodiments, each of the antibody 1 is independently selected from antibodies that specifically bind to amino acids at positions 223-242 or positions 262-281 of cTnT. In some embodiments, the antibody 1 is an antibody that specifically binds to amino acids at positions 223-242 of cTnT.

In some embodiments, each of the antibody 3 is independently selected from antibodies that specifically bind to amino acids at positions 67-86, positions 119-138, positions 132-151, positions 145-164 or positions 171-190 of cTnT. In some embodiments, each of the antibody 3 is independently selected from antibodies that specifically bind to amino acids at positions 119-138 or positions 132-151 of cTnT.

In some embodiments, each of the antibody 5 is independently selected from antibodies that specifically bind to amino acids at positions 1-15, positions 13-22, positions 18-22, positions 18-28, positions 18-35, positions 22-31, positions 22-40, positions 23-29, positions 24-40, positions 25-40, positions 26-35, positions 34-37, positions 41-49, positions 83-89, positions 86-90, positions 87-90, positions 117-126, positions 130-145, positions 169-178, positions 186-192, positions 190-196 or positions 195-209 of cTnI. In some embodiments, each of the antibody 5 is independently selected from antibodies that specifically bind to amino acids at positions 22-40, at positions 41-49 or positions 83-89 of cTnI. In some embodiments, each of the antibody 5 is independently selected from antibodies that specifically bind to amino acids at positions 41-49 of cTnI.

In some embodiments, the capture antibody is the first group of antibodies and the detection antibody is the second group of antibodies. Studies have found that the detection of total cTnITC demonstrates a higher signal-to-noise ratio when the first group of antibodies is used as the capture antibody and the second group of antibodies is used as the detection antibody.

In the disclosure, commercially available antibodies targeting specific epitopes may be selected, or antibodies in the disclosure that have specific binding activity for specific epitopes of different forms of cTnITC may be obtained by means of general technical means in the art. One exemplary embodiment includes obtaining a monoclonal antibody with specific binding activity by hybridoma technology, which specifically includes the steps of selection and preparation of two parental cell lines, cell fusion, selective culture and cloning of hybridoma cells, preparation of monoclonal antibodies, specificity identification, purification, etc. Further, the steps of epitope identification and screening may be included, such as the identification of antibody-binding epitopes using techniques such as ELISA or surface plasmon resonance (SPR).

Use

Due to the low sensitivity of existing cTnITC detection kits, the use thereof in the diagnosis of myocardial injury is severely compromised. Clinically, cTnI and cTnT are also commonly selected as highly specific and highly sensitive markers for myocardial infarction. On the basis of the above-mentioned composition, kit and detection method, the disclosure enables the rapid, highly sensitive and highly specific detection of total cTnITC, which facilitates an in-depth understanding of the correlation between troponin and myocardial injury. Studies have shown that total cTnITC is more sensitive to myocardial injury than cTnI and cTnT, and thus can serve as a more sensitive marker for myocardial injury to aid in disease diagnosis and prognosis assessment.

In a fourth aspect, the disclosure provides the use of the composition according to any of the first aspect in the preparation of a reagent for detecting a cardiac troponin ternary complex.

In a fifth aspect, the disclosure provides the use of the composition according to any of the first aspect, the kit according to any of the second aspect, or the method according to any of the third aspect in the *in-vitro* diagnosis of myocardial injury.

In a sixth aspect, the disclosure provides a method for diagnosing myocardial injury *in vitro,* which method includes a step of detecting a cardiac troponin ternary complex in a sample from a subject using the composition according to any of the first aspect, the kit according to any of the second aspect, or the method according to any of the third aspect.

In some embodiments, the sample is body fluid or tissue sample from a subject, such as whole blood, serum, plasma (including heparin lithium plasma and EDTA plasma), urine, saliva, or biological tissue or cells, preferably whole blood, serum or plasma.

Beneficial Effects

1) The disclosure achieves the rapid, highly sensitive and highly specific detection of total cTnITC. In the disclosure, an antibody that specifically binds to TnC is used as the capture antibody or detection antibody, and a series of experiments have demonstrated that the use of the TnC antibody may significantly increase the signal-to-noise ratio of the system and improve the sensitivity of the system.

2) Since the existing forms of troponin in the blood are associated with the physiological and pathological status of an individual, the specific detection of the cardiac troponin ternary complex cTnITC facilitates an in-depth understanding of the correlation between troponin and diseases. The total concentration of a cardiac troponin ternary complex cTnITC exhibits a consistent trend of change with cTnT and cTnI, and thus has clinical value; the total concentration of cTnITC exhibits a faster rate of change, is more sensitive to myocardial injury in patients, and thus can serve as a more sensitive myocardial injury marker to aid in disease diagnosis and prognosis assessment.

3) The detection kit of the disclosure is used in combination with a fully automated chemiluminescence analyser, which enables automated, fast and high-throughput detection of the cardiac troponin ternary complex cTnITC.

4) The detection kit of the disclosure has high sensitivity, excellent specificity and good reproducibility.

Examples

Example 1 Detection Method and Construction of Detection Kits

The capture and detection antibodies were applied to a double-antibody sandwich chemiluminescence immunoassay, and detection kits were constructed for detecting a total cardiac troponin ternary complex cTnITC in a sample.

Specifically, the following kits were constructed for subsequent experiments:

1. Total cTnITC detection kit 1:

Capture antibody: antibody 1: 7E7 (which specifically binds to the amino acid fragment 223-242 of cTnT); and antibody 3: 329cc (which specifically binds to the amino acid fragment 119-138 of cTnT).
Detection antibody: antibody 4: 20C6cc (which specifically binds to an epitope of the cTnIC complex).

2. Total cTnITC detection kit 2:

Capture antibody: antibody 1: 7E7 (which specifically binds to the amino acid fragment 223-242 of cTnT); and antibody 3: 329cc (which specifically binds to the amino acid fragment 119-138 of cTnT).
Detection antibody: antibody 5: 19C7cc (which specifically binds to the amino acid fragment 41-49 of cTnI).

3. Total cTnITC detection kit 3:

Capture antibody: antibody 1: 7E7 (which specifically binds to the amino acid fragment 223-242 of cTnT); and antibody 3: 329cc (which specifically binds to the amino acid fragment 119-138 of cTnT).
Detection antibody: antibody 2: 7B9cc (which specifically binds to TnC); and antibody 4: 20C6cc (which specifically binds to an epitope of the cTnIC complex).

4. Total cTnITC detection kit 4:

Capture antibody: antibody 1: 7E7 (which specifically binds to the amino acid fragment 223-242 of cTnT); and antibody 3: 329cc (which specifically binds to the amino acid fragment 119-138 of cTnT).
Detection antibody: antibody 2: 7B9cc (which specifically binds to TnC); and antibody 5: 19C7cc (which specifically binds to the amino acid fragment 41-49 of cTnI).

5. Total cTnITC detection kit 5:

Capture antibody: antibody 1: 7E7 (which specifically binds to the amino acid fragment 223-242 of cTnT); and antibody 3: 329cc (which specifically binds to the amino acid fragment 119-138 of cTnT).
Detection antibody: antibody 2: 7B9cc (which specifically binds to TnC).

6. Total cTnITC detection kit 6:

Capture antibody: antibody 1: 7E7 (which specifically binds to the amino acid fragment 223-242 of cTnT).
Detection antibody: antibody 2: 7B9cc (which specifically binds to TnC).

7. Total cTnITC detection kit 7:

Capture antibody: antibody 1: 155 (which specifically binds to the amino acid fragment 262-281 of cTnT).
Detection antibody: antibody 2: 7B9cc (which specifically binds to TnC); and antibody 4: Tcom8 (which specifically binds to an epitope of the cTnIC complex).

8. Total cTnITC detection kit 8:

Capture antibody: antibody 1: 7E7 (which specifically binds to the amino acid fragment 223-242 of cTnT); and antibody 3: 406cc (which specifically binds to the amino acid fragment 132-151 of cTnT).
Detection antibody: antibody 2: 7B9cc (which specifically binds to TnC); and antibody 4: 20C6cc (which specifically binds to an epitope of the cTnIC complex).

9. Total cTnITC detection kit 9:

Capture antibody: antibody 1: 7E7 (which specifically binds to the amino acid fragment 223-242 of cTnT); and antibody 3: 300cc (which specifically binds to the amino acid fragment 119-138 of cTnT).
Detection antibody: antibody 2: 7B9cc (which specifically binds to TnC); and antibody 4: 20C6cc (which specifically binds to an epitope of the cTnIC complex).

10. Total cTnITC detection kit 10:

Capture antibody: antibody 1: 155 (which specifically binds to the amino acid fragment 262-281 of cTnT); and antibody 3: 406cc (which specifically binds to the amino acid fragment 132-151 of cTnT).
Detection antibody: antibody 2: 7B9cc (which specifically binds to TnC); and antibody 4: 20C6cc (which specifically binds to an epitope of the cTnIC complex).

11. Total cTnITC detection kit 11:

Capture antibody: antibody 1: 7E7 (which specifically binds to the amino acid fragment 223-242 of cTnT); and antibody 3: 7G7 (which specifically binds to the amino acid fragment 67-86 of cTnT).
Detection antibody: antibody 2: 7B9cc (which specifically binds to TnC); and antibody 4: 20C6cc (which specifically binds to an epitope of the cTnIC complex).

12. Total cTnITC detection kit 12:

Capture antibody: antibody 1: 7E7 (which specifically binds to the amino acid fragment 223-242 of cTnT); and antibody 3: 1C11cc (which specifically binds to the amino acid fragment 171-190 of cTnT).
Detection antibody: antibody 2: 7B9cc (which specifically binds to TnC); and antibody 4: Tcom8 (which specifically binds to an epitope of the cTnIC complex).

In the experiments, kits were also used in which antibody 3, employed in the aforementioned total cTnITC detection kits 3-5 and 8-12, was replaced with the following antibodies: antibody 7F4 with a specific binding site at the amino acid fragment 67-86 of cTnT, and antibody 2F3, 1A11 or 1F11cc with a specific binding site at the amino acid fragment 145-164 of cTnT. In the experiments, kits were also used in which antibody 5, employed in the aforementioned total cTnITC detection kit 4, was replaced with the following antibodies: antibody M18cc with a specific binding site at the amino acid fragment 18-28 of cTnI, antibody 16A11cc, 16A12cc or 8E10cc with a specific binding site at the amino acid fragment 86-90 of cTnI, antibody M46 with a specific binding site at the amino acid fragment 130-145 of cTnI, and antibody MF4cc with a specific binding site at the amino acid fragment 190-196 of cTnI.

The total cTnITC detection kits include:

A. Magnetic bead coating working solution, for the capture of a cardiac troponin ternary complex cTnITC in a sample. The magnetic bead coating working solution includes: a mixture of superparamagnetic microparticles coated with capture antibodies.

B. Enzyme conjugate working solution, for the detection of the cardiac troponin ternary complex cTnITC antigen captured by the superparamagnetic microparticles. The enzyme conjugate working solution includes: an alkaline phosphatase-labelled detection antibody.

The detection method was as follows:

Step 1: A sample was added to a reaction tube together with a magnetic bead coating working solution and an enzyme conjugate working solution. After incubation, a cardiac troponin ternary complex cTnITC in the sample was bound to an antibody coated on the magnetic beads, and meanwhile, an antibody-alkaline phosphatase conjugate was bound to the cardiac troponin ternary complex cTnITC in the sample. After the reaction was completed, the solid phase was placed in a magnetic field. The magnetic field attracted the magnetic beads. The substance bound to the solid phase was retained, and the unbound substances were washed away.

Step 2: A chemiluminescent substrate (3-(2-spiroadamantane)-4-methoxy-4-(3-phosphoryloxy)-phenyl-1,2-dioxetane, AMPPD) was added to the reaction tube, and decomposed by the alkaline phosphatase, with one phosphate group removed, to form an unstable intermediate product. The intermediate product underwent intramolecular electron transfer to produce a methyl m-oxybenzoate anion. When the methyl m-oxybenzoate anion in the excited state returned to the ground state, chemiluminescence was produced. The number of photons produced during the reaction was then measured using a photomultiplier tube. The number of photons produced was proportional to the concentration of the ternary cTnITC complex in the sample. The amount of an analyte in the sample was determined by a calibration curve.

The above total cTnITC detection kits were designed for use in combination with Mindray fully automated chemiluminescence analysers, including models such as CL2000i, CL6000i and CL8000i.

Example 2 Signal-to-Noise Ratio Analysis of Total cTnITC Detection Kits

Samples containing different concentrations of antigens were prepared, including two high-concentration samples (high-value sample 1 and high-value sample 2) and two low-concentration samples (low-value sample 1 and low-value sample 2). The antigen was a recombinant cardiac troponin ternary complex cTnITC (Hytest, 8ITCR). The samples were separately analysed using total cTnITC detection kits 1-12. Meanwhile, the signal from the blank sample without antigen was recorded, and the signal-to-noise ratio was calculated.

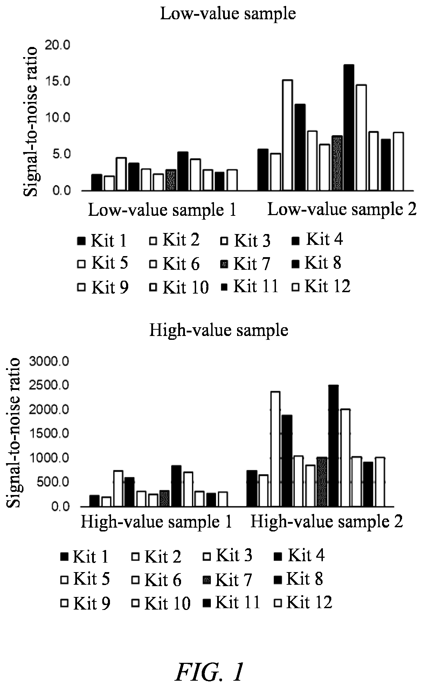

The test results are shown in FIG. 1. The signal-to-noise ratio of detection kit 5 was higher than that of detection kit 6, indicating that the addition of antibody 3 that specifically binds to the amino acid fragment 67-222 of cTnT increased the signal-to-noise ratio. The signal-to-noise ratio of detection kit 3 was significantly higher than those of detection kit 1 and detection kit 5, and the signal-to-noise ratio of detection kit 4 was significantly higher than those of detection kit 2 and detection kit 5, indicating that the combined use of antibody 2 that specifically binds to TnC with antibody 4 or antibody 5 was able to significantly increase the signal-to-noise ratio. The signal-to-noise ratios of detection kits 3, 8 and 9 were similar, indicating that selecting antibodies that specifically bind to different fragments of amino acids at positions 67-222 of cTnT as antibody 3 resulted in similar signal-to-noise ratios in the resulting kits. The signal-to-noise ratio of detection kit 8 was significantly superior to that of detection kit 10, indicating that selecting an antibody that specifically binds to amino acids at positions 223-242 of cTnT as antibody 1 resulted in a higher signal-to-noise ratio than selecting an antibody that specifically binds to amino acids at positions 262-281 of cTnT. The signal-to-noise ratio of detection kit 3 was significantly higher than those of detection kits 11 and 12, indicating that selecting an antibody that specifically binds to amino acids at positions 119-13 of cTnT 8 as antibody 3 was more advantageous than selecting an antibody that specifically binds to amino acids at positions 67-86 or positions 171-190 of cTnT.

In addition, replacing antibody 3 in the total cTnITC detection kits 3-5 and 8-12 with the following antibodies: antibody 7F4 with a specific binding site at the amino acid fragment 67-86 of cTnT, and antibody 2F3, 1A11 or 1F11cc with a specific binding site at the amino acid fragment 145-164 of cTnT, and replacing antibody 5 used in the total cTnITC detection kit 4 with the following antibodies: antibody M18cc with a specific binding site at the amino acid fragment 18-28 of cTnI, antibody 16A11cc, 16A12cc or 8E10cc with a specific binding site at the amino acid fragment 86-90 of cTnI, antibody M46 with a specific binding site at the amino acid fragment 130-145 of cTnI, and antibody MF4cc with a specific binding site at the amino acid fragment 190-196 of cTnI, both effectively reflecting signal differences among samples. The antibodies herein were purchased from HyTest Biotech (Shanghai) Co., Ltd.

Example 3 Specificity Analysis of Total cTnITC Detection Kits

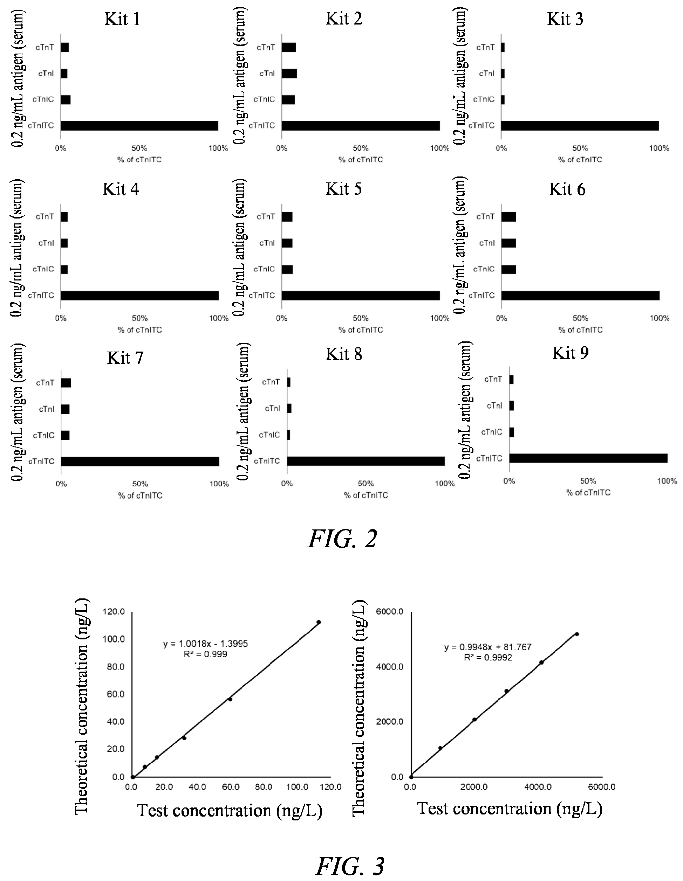

Equal concentrations of different antigens were spiked into the serum of healthy people, and the samples were separately analysed using total cTnITC detection kits 1-9 by the chemiluminescent immunoassay method. The antigens analysed included: cTnT (Hytest, 8RTT5), cTnI (Hytest, 8RT17), cTnIC (Hytest, 8ICR3) and cTnITC (Hytest, 8ITCR). The experimental results are shown in FIG. 2. Detection kits 1-9 for the ternary troponin complex cTnITC were able to recognise only the cTnITC antigen, but were unable to recognise cTnT, cTnI or the binary cTnIC complex.

Replacing antibody 3 in the total cTnITC detection kits 3-5 and 8-12 with the following antibodies: antibody 7F4 with a specific binding site at the amino acid fragment 67-86 of cTnT, and antibody 2F3, 1A11 or 1F11cc with a specific binding site at the amino acid fragment 145-164 of cTnT, and replacing antibody 5 used in the total cTnITC detection kit 4 with the following antibodies: antibody M18cc with a specific binding site at the amino acid fragment 18-28 of cTnI, antibody 16A11cc, 16A12cc or 8E10cc with a specific binding site at the amino acid fragment 86-90 of cTnI, antibody M46 with a specific binding site at the amino acid fragment 130-145 of cTnI, and antibody MF4cc with a specific binding site at the amino acid fragment 190-196 of cTnI, both effectively recognising the cTnITC antigen.

Example 4 Establishment of Limit of Blank and Limit of Detection for Total cTnITC Detection Kits

The limit of blank (LoB) and limit of detection (LoD) were established according to the recommendations of the Clinical and Laboratory Standards Institute (CLSI) (EP-17A2 Protocols for Determination of Limits of Detection and Limits of Quantitation).

The results of LoB test were derived from 5 blank samples, which were run over 4 days with 4 replicates per assay.

$$\text{General formula: LoB} = \text{mean} + 1.65*\text{SD}$$

The results of LoD test were derived from 5 low-concentration samples, which were run over 4 days with 4 replicates per assay.

$$\text{General formula: LoD} = \text{LoB} + 1.65*\text{SD}$$

The limit of blank and limit of detection were established for kits 1-9. The test results are shown in Table 1.

Table 1: Limits of blank (LoB) and limits of detection (LoD) of the kits

| Total cTnITC detection kit | 1 | 2 | 3 | 4 | 5 | 6 | 7 | 8 | 9 |
|---|---|---|---|---|---|---|---|---|---|
| Limits of blank (LoB) (ng/L) | 4.8 | 6.1 | 0.9 | 2.3 | 3.2 | 4.2 | 3.4 | 0.8 | 1.8 |
| Limit of detection (LoD) (ng/L) | 8.6 | 11.2 | 1.5 | 3.8 | 5.8 | 7.2 | 5.9 | 1.4 | 3.1 |

The limit of blank and limit of detection of detection kit 5 were lower than those of detection kit 6, indicating that the addition of antibody 3 that specifically binds to the amino acid fragment 119-138 of cTnT improved the sensitivity. The limit of blank and limit of detection of detection kit 3 were significantly lower than those of detection kit 1 and detection kit 5, and the limit of blank and limit of detection of detection kit 4 were significantly lower than those of detection kit 2 and detection kit 5, indicating that the use of antibody 2 that specifically binds to TnC in combination with antibody 4 or antibody 5 significantly improved the sensitivity of the kits.

In addition to the above kits, replacing antibody 3 used in the total cTnITC detection kits 3-5 and 8-12 with the following antibodies: antibody 7F4 with a specific binding site at the amino acid fragment 67-86 of cTnT, and antibody 2F3, 1A11 or 1F11cc with a specific binding site at the amino acid fragment 145-164 of cTnT, and replacing antibody 5 used in the total cTnITC detection kit 4 with the following antibodies: antibody M18cc with a specific binding site at the amino acid fragment 18-28 of cTnI, antibody 16A11cc, 16A12cc or 8E10cc with a specific binding site at the amino acid fragment 86-90 of cTnI, antibody M46 with a specific binding site at the amino acid fragment 130-145 of cTnI, and antibody MF4cc with a specific binding site at the amino acid fragment 190-196 of cTnI, both resulting in low LoB and LoD values.

Example 5 Linear Analysis of Total cTnITC Detection Kits

Detection kit 3 and detection kit 4 were selected for linear analysis due to a high signal-to-noise ratio and a low limit of detection thereof.

Two clinical serum samples with different concentrations were selected as high-concentration samples. The high-concentration samples were diluted proportionally to produce a series of diluted samples with concentration ranges of 0-120 ng/L and 0-6000 ng/L. The samples were separately analysed using detection kit 3 by the chemiluminescent immunoassay method. The average of the test concentration results was linearly fitted against the theoretical concentration, and the correlation coefficient within the linear range was calculated. The experimental results are shown in FIG. 3. The test concentrations of the diluted samples exhibited a linear relationship with the theoretical concentration. Within the linear range, the R2 value for the low-concentration range (0-120 ng/L) was 0.9990, and the R2 value for the high-concentration range (0-6000 ng/L) was 0.9992.

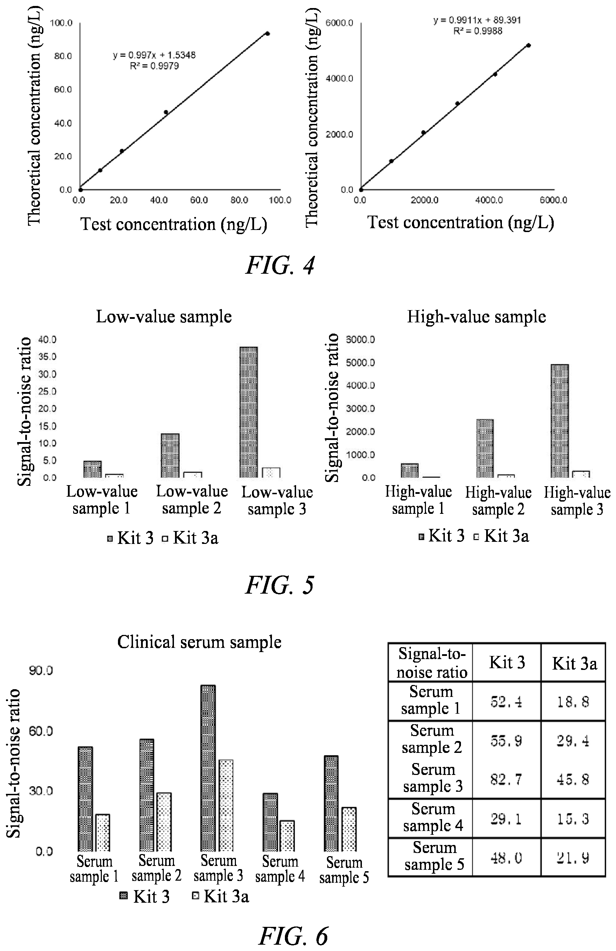

The samples were separately analysed using detection kit 4 by the chemiluminescent immunoassay method. The average of the test concentration results was linearly fitted against the theoretical concentration, and the correlation coefficient within the linear range was calculated. The experimental results are shown in FIG. 4. The test concentrations of the diluted samples exhibited a linear relationship with the theoretical concentration. Within the linear range, the R2 value for the low-concentration range (0-120 ng/L) was 0.9979, and the R2 value for the high-concentration range (0-6000 ng/L) was 0.9988.

Example 6 Construction and Testing of Kit with Capture Antibody and Detection Antibody Exchanged

Detection kit 3a was constructed by exchanging the capture antibody and the detection antibody of detection kit 3.

Detection kit 3 includes:

Capture antibody: antibody 1: 7E7 (which specifically binds to the amino acid fragment 223-242 of cTnT); and antibody 3: 329cc (which specifically binds to the amino acid fragment 119-138 of cTnT).
Detection antibody: antibody 2: 7B9cc (which specifically binds to TnC); and antibody 4: 20C6cc (which specifically binds to epitopes of the cTnIC complex).

Detection kit 3a includes:

Capture antibody: antibody 2: 7B9cc (which specifically binds to TnC); and antibody 4: 20C6cc (which specifically binds to epitopes of the cTnIC complex).
Detection antibody: antibody 1: 7E7 (which specifically binds to the amino acid fragment 223-242 of cTnT); and antibody 3: 329cc (which specifically binds to the amino acid fragment 119-138 of cTnT).

Samples containing different concentrations of antigens were prepared, including three high-concentration samples and three low-concentration samples. The antigen was a recombinant cardiac troponin ternary complex (Hytest, 8ITCR). The samples were separately analysed using detection kits 3 and 3a. Meanwhile, the signal from the blank sample without antigen was recorded, and the signal-to-noise ratio was calculated. The test results are shown in FIG. 5, which showed that the signal-to-noise ratio decreased after the detection antibody and the capture antibody of detection kit 3 were exchanged.

Clinical serum samples from patients with cardiovascular diseases were separately analysed using detection kits 3 and 3a. Meanwhile, the signals from serum samples of healthy people were recorded, and the signal-to-noise ratio was calculated. The test results are shown in FIG. 6. The signal-to-noise ratio of detection kit 3 was significantly higher than that of detection kit 3a, indicating that when the preferred capture antibodies and detection antibodies were as follows: Capture antibody: antibody 1 (which specifically binds to the amino acid fragment 223-242 of cTnT) and antibody 3 (which specifically binds to the amino acid fragment 119-138 of cTnT); detection antibodies: antibody 2 (which specifically binds to TnC) and antibody 4 (which specifically binds to epitopes of the cTnIC complex), the kit had a higher signal-to-noise ratio. The signal-to-noise ratio of detection kit 3a was lower than that of detection kit 3, while the variation pattern of signal differences of detection kit 3a across different samples was similar to that of detection kit 3.

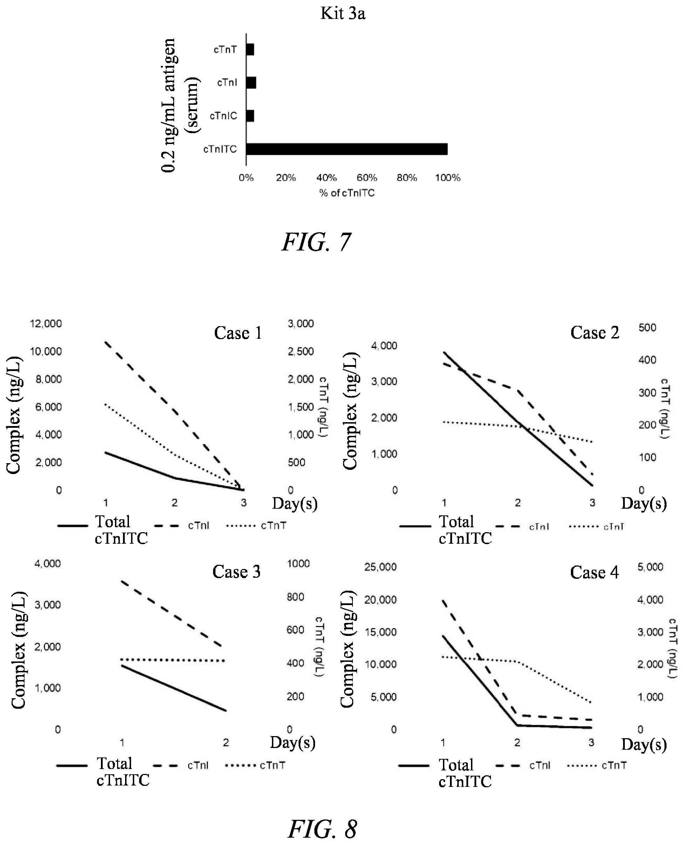

The specificity of detection kit 3a was analysed. Equal concentrations of different antigens were spiked into the serum of healthy people, and the samples were analysed using cardiac troponin ternary complex cTnITC detection kit 3a by the chemiluminescent immunoassay method. The antigens analysed included: cTnT (Hytest, 8RTT5), cTnI (Hytest, 8RT17), cTnIC (Hytest, 8ICR3) and cTnITC (Hytest, 8ITCR). The experimental results are shown in FIG. 7. Detection kit 3a for the cardiac troponin ternary complex cTnITC was able to recognise the cTnITC antigen, but was unable to recognise cTnT, cTnI or the binary cTnIC complex.

In summary, detection kit 3a can be used for the detection of clinical serum samples, while detection kit 3 is preferred with superior performance in terms of overall sensitivity and specificity.

Example 7 Clinical Performance Evaluation of Total cTnITC-Specific Immunoassay

Total cTnITC detection kit 3 was used for clinical performance evaluation.

To evaluate the clinical performance of the sandwich immunoassay for the cardiac troponin ternary complex cTnITC, dynamic monitoring was performed on serum samples from patients at different time points. Meanwhile, the high-sensitivity cTnI and cTnT levels in the serum of patients were detected.

Patients with myocardial damage were enrolled, and the enrolled patients were aged 18 years or older. These patients were diagnosed with myocardial infarction or heart failure, or had undergone cardiac surgery, and exhibited elevated levels of high-sensitivity cTnI or high-sensitivity cTnT. After enrollment, heparin lithium plasma samples from the patients were collected for three consecutive days, and the concentrations of total cTnITC in the samples were measured using a Mindray chemiluminescence analyser and accompanying reagents.

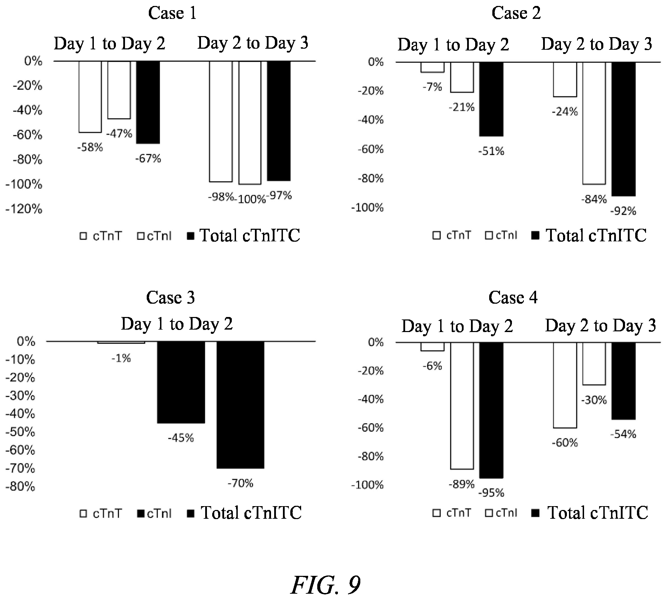

The results are shown in FIG. 8. The concentration of total cTnITC exhibited a consistent trend of change with the high-sensitivity cTnI and cTnT levels, indicating that the concentration of total cTnITC had clinical value and could be used for the evaluation of myocardial damage in patients. A further analysis was performed on the concentration changes of total cTnITC. As shown in FIG. 9, the concentration of total cTnITC exhibited a faster rate of change compared with cTnI and cTnT, indicating that the concentration changes of total cTnITC were more sensitive to myocardial damage in patients.

Various modifications of the disclosure in addition to those described herein will be apparent to those skilled in the art from the foregoing description. Such modifications are also intended to fall within the scope of the appended claims. The full scope of the disclosure is given by the appended claims and any equivalents thereof.

**Claims**

1. A composition for detecting a cardiac troponin ternary complex in a sample, comprising a first group of antibodies and a second group of antibodies, wherein

    the first group of antibodies comprises one or more antibody 1, each being independently selected from antibodies that specifically bind to any one segment of the amino acid sequence of positions 223-287 of cTnT (cardiac troponin T);
    the second group of antibodies comprises one or more antibody 2, each being independently selected from antibodies that specifically bind to any one segment of the amino acid sequence of TnC (troponin C).

2. The composition of claim 1, wherein the first group of antibodies further comprises one or more antibody 3, each being independently selected from antibodies that specifically bind to any one segment of the amino acid sequence of positions 67-222 of cTnT.

3. The composition of claim 1 or 2, wherein the second group of antibodies further comprises:

    one or more antibody 4, each being independently selected from antibodies that specifically bind to cTnIC (cardiac troponin IC binary complex); and/or
    one or more antibody 5, each being independently selected from antibodies that specifically bind to any one segment of the amino acid sequence of positions 18-210 of cTnI.

4. The composition of claim 3, wherein the first group of antibodies comprises one or more of the antibody 1 and one or more of the antibody 3; and the second group of antibodies comprises one or more of the antibody 2, one or more of the antibody 4 and/or one or more of the antibody 5.

5. The composition of any one of claims 1-4, wherein each of the antibody 1 is independently selected from antibodies that specifically bind to amino acids at positions 223-242 or positions 262-281 of cTnT; preferably, antibodies that specifically bind to amino acids at positions 223-242 of cTnT.

6. The composition of any one of claims 2-5, wherein each of the antibody 3 is independently selected from antibodies that specifically bind to amino acids at positions 67-86, positions 119-138, positions 132-151, positions 145-164 or positions 171-190 of cTnT;
    preferably, each of the antibody 3 is independently selected from antibodies that specifically bind to amino acids at positions 119-138 or positions 132-151 of cTnT.

7. The composition of any one of claims 3-6, wherein each of the antibody 5 is independently selected from antibodies that specifically bind to amino acids at positions 1-15, positions 13-22, positions 18-22, positions 18-28, positions 18-35, positions 22-31, positions 22-40, positions 23-29, positions 24-40, positions 25-40, positions 26-35, positions 34-37, positions 41-49, positions 83-89, positions 86-90, positions 87-90, positions 117-126, positions 130-145, positions 169-178, positions 186-192, positions 190-196 or positions 195-209 of cTnI;

preferably, each of the antibody 5 is independently selected from antibodies that specifically bind to amino acids at positions 22-40, positions 41-49 or positions 83-89 of cTnI;

more preferably, each of the antibody 5 is independently selected from antibodies that specifically bind to amino acids at positions 41-49 of cTnI.

8. A kit for detecting a cardiac troponin ternary complex in a sample, comprising a capture antibody and a detection antibody, wherein the capture antibody is selected from one group of a first group of antibodies and a second group of antibodies, and the detection antibody is selected from the other group of the first group of antibodies and the second group of antibodies, wherein

the first group of antibodies comprises one or more antibody 1, each being independently selected from antibodies that specifically bind to any one segment of the amino acid sequence of positions 223-287 of cTnT; the second group of antibodies comprises one or more antibody 2, each being independently selected from antibodies that specifically bind to any one segment of the amino acid sequence of TnC.

9. The kit of claim 8, wherein the first group of antibodies further comprises one or more antibody 3, each being independently selected from antibodies that specifically bind to any one segment of the amino acid sequence of positions 67-222 of cTnT.

10. The kit of claim 8 or 9, wherein the second group of antibodies further comprises:

one or more antibody 4, each being independently selected from antibodies that specifically bind to cTnIC; and/or one or more antibody 5, each being independently selected from antibodies that specifically bind to any one segment of the amino acid sequence of positions 18-210 of cTnI.

11. The kit of any one of claims 8-10, wherein the first group of antibodies comprises one or more of the antibody 1 and one or more of the antibody 3; the second group of antibodies comprises one or more of the antibody 2, one or more of the antibody 4 and/or one or more of the antibody 5.

12. The kit of any one of claims 8-11, wherein the capture antibody is the first group of antibodies and the detection antibody is the second group of antibodies.

13. The kit of any one of claims 8-12, wherein each of the antibody 1 is independently selected from antibodies that specifically bind to amino acids at positions 223-242 or positions 262-281 of cTnT, preferably antibodies that specifically bind to amino acids at positions 223-242 of cTnT; and/or

each of the antibody 3 is independently selected from antibodies that specifically bind to amino acids at positions 67-86, positions 119-138, positions 132-151, positions 145-164 or positions 171-190 of cTnT; preferably, each of the antibody 3 is independently selected from antibodies that specifically bind to amino acids at positions 119-138 or 132-151 of cTnT; and/or each of the antibody 5 is independently selected from antibodies that specifically bind to amino acids at positions 1-15, positions 13-22, positions 18-22, positions 18-28, positions 18-35, positions 22-31, positions 22-40, positions 23-29, positions 24-40, positions 25-40, positions 26-35, positions 34-37, positions 41-49, positions 83-89, positions 86-90, positions 87-90, positions 117-126, positions 130-145, positions 169-178, positions 186-192, positions 190-196 or positions 195-209 of cTnI; preferably, each of the antibody 5 is independently selected from antibodies that specifically bind to amino acids at positions 22-40, positions 41-49 or positions 83-89 of cTnI; more preferably, each of the antibody 5 is independently selected from antibodies that specifically bind to amino acids at positions 41-49 of cTnI.

14. A method for detecting a cardiac troponin ternary complex in a sample *in vitro,* comprising steps of:

obtaining a test sample; contacting the test sample with a capture antibody to form an antibody-antigen complex; contacting the antibody-antigen complex with a detection antibody bearing a detectable label to form an antibody-antigen-antibody complex; and detecting the signal produced by the detectable label to determine the presence and/or total amount of a cardiac troponin ternary complex in the test sample, wherein the capture antibody is selected from one group of a first group of antibodies and a second group of antibodies, and the detection antibody is selected from the other group of the first group of antibodies and the second group of antibodies:

the first group of antibodies includes one or more antibody 1, each being independently selected from antibodies that specifically bind to any one segment of the amino acid sequence of positions 223-287 of cTnT; the second group of antibodies includes one or more antibody 2, each being independently selected from antibodies that specifically bind to any one segment of the amino acid sequence of TnC.

15. The method of claim 14, wherein the first group of antibodies further comprises one or more antibody 3, each being independently selected from antibodies that specifically bind to any one segment of the amino acid sequence of positions 67-222 of cTnT.

16. The method of claim 14 or 15, wherein the second group of antibodies further comprises:

one or more antibody 4, each being independently selected from antibodies that specifically bind to cTnIC; and/or one or more antibody 5, each being independently selected from antibodies that specifically bind to any one segment of the amino acid sequence of positions 18-210 of cTnI.

17. The method of any one of claims 14-16, wherein the first group of antibodies comprises one or more of the antibody 1 and one or more of the antibody 3; the second group of antibodies comprises one or more of the antibody 2, one or more of the antibody 4 and/or one or more of the antibody 5.

18. The method of any one of claims 14-17, wherein the capture antibody is the first group of antibodies and the detection antibody is the second group of antibodies.

19. Use of the composition of any one of claims 1-7 in the preparation of a reagent for detecting total cardiac troponin ternary complexes.

20. Use of the composition of any one of claims 1-7, the kit of any one of claims 8-13, or the method of any one of claims 14-18 in the *in-vitro* diagnosis of myocardial injury.

21. A method for diagnosing myocardial injury *in vitro,* comprising a step of detecting total cardiac troponin ternary complexes in a sample from a subject using the composition of any one of claims 1-7, the kit of any one of claims 8-13, or the method of any one of claims 14-18.

Low-value sample

High-value sample

*FIG. 1*

*FIG. 2*

*FIG. 3*

*FIG. 4*

*FIG. 5*

| Signal-to-noise ratio | Kit 3 | Kit 3a |
|---|---|---|
| Serum sample 1 | 52.4 | 18.8 |
| Serum sample 2 | 55.9 | 29.4 |
| Serum sample 3 | 82.7 | 45.8 |
| Serum sample 4 | 29.1 | 15.3 |
| Serum sample 5 | 48.0 | 21.9 |

*FIG. 6*

FIG. 7

FIG. 8

*FIG. 9*

## INTERNATIONAL SEARCH REPORT

| International application No. |
| --- |
| **PCT/CN2023/112993** |

| A. | CLASSIFICATION OF SUBJECT MATTER |
| --- | --- |

G01N33/68(2006.01)i

According to International Patent Classification (IPC) or to both national classification and IPC

| B. | FIELDS SEARCHED |
| --- | --- |

Minimum documentation searched (classification system followed by classification symbols)

IPC： G01N

Documentation searched other than minimum documentation to the extent that such documents are included in the fields searched

Electronic data base consulted during the international search (name of data base and, where practicable, search terms used)

CNTXT: ENTXTC; WPABSC; VEN; ENTXT; CNKI; Web of Science; 百度学术, BAIDU SCHOLAR: 深圳迈瑞, 心肌肌钙蛋白, 肌钙蛋白, 复合物, cTnT, TnC, TnI, 抗体, 标记, 7E7, 329cc, 7B9cc, cardiac troponin, troponin, complex, antibod+, label

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| X | US 2009233268 A1 (AXELA INC.) 17 September 2009 (2009-09-17) description, paragraphs [0006]-[0094] | 1-21 |
| A | CN 114994304 A (XIAMEN BIOTIME BIOTECHNOLOGY CO., LTD. et al.) 02 September 2022 (2022-09-02) entire document | 1-21 |
| A | US 5795725 A (BIOSITE DIAGNOSTICS INC.) 18 August 1998 (1998-08-18) entire document | 1-21 |
| A | US 6174686 B1 (BIOSITE DIAGNOSTICS INC.) 16 January 2001 (2001-01-16) entire document | 1-21 |
| A | US 2004219604 A1 (UNIVERSITY OF TURKU) 04 November 2004 (2004-11-04) entire document | 1-21 |
| A | US 6156521 A (BIOSITE DIAGNOSTICS INC.) 05 December 2000 (2000-12-05) entire document | 1-21 |
| A | US 2005164317 A1 (BIOSITE INC.) 28 July 2005 (2005-07-28) entire document | 1-21 |

☐ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
| --- | --- |
| **07 December 2023** | **11 December 2023** |

| Name and mailing address of the ISA/CN | Authorized officer |
| --- | --- |
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**
Information on patent family members

International application No.

**PCT/CN2023/112993**

| Patent document cited in search report | | | Publication date (day/month/year) | Patent family member(s) | | | Publication date (day/month/year) |
|---|---|---|---|---|---|---|---|
| US | 2009233268 | A1 | 17 September 2009 | US | 8338189 | B2 | 25 December 2012 |
| CN | 114994304 | A | 02 September 2022 | None | | | |
| US | 5795725 | A | 18 August 1998 | None | | | |
| US | 6174686 | B1 | 16 January 2001 | None | | | |
| US | 2004219604 | A1 | 04 November 2004 | US | 7348157 | B2 | 25 March 2008 |
| US | 6156521 | A | 05 December 2000 | None | | | |
| US | 2005164317 | A1 | 28 July 2005 | US | 7604946 | B2 | 20 October 2009 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **HARLOW** ; **LANE**. Antibodies: A Laboratory Manual. Cold Spring Harbor Laboratory Press, 1988
- **HARLOW** ; **LANE**. Using Antibodies: A Laboratory Manual. Cold Spring Harbor Laboratory Press, 1999
- **ANDERSON**. *Circulation Research*, 1995, vol. 76 (4), 681-686
- **FERRIERES**. *Clinical Chemistry*, 1998, vol. 44, 487-493
- **KABAT**. Sequences of Proteins of Immunological Interest. National Institutes of Health, 1987
- **CHOTHIA** ; **LESK**. *J. Mol. Biol.*, 1987, vol. 196, 901-917
- **CHOTHIA et al.** *Nature*, 1989, vol. 342, 878-883